Europäisches Patentamt

European Patent Office

Office européen des brevets

(19)

(11) **EP 1 076 575 B1**

(12) **FASCICULE DE BREVET EUROPEEN**

(45) Date de publication et mention
de la délivrance du brevet:
**22.10.2003 Bulletin 2003/43**

(51) Int Cl.⁷: **A61M 5/32**

(86) Numéro de dépôt international:
**PCT/FR99/01083**

(21) Numéro de dépôt: **99918024.3**

(22) Date de dépôt: **07.05.1999**

(87) Numéro de publication internationale:
**WO 99/059660 (25.11.1999 Gazette 1999/47)**

(54) **DISPOSITIF DE PROTECTION ET DE NEUTRALISATION D'UNE AIGUILLE A USAGE MEDICAL**

VORRICHTUNG ZUM SCHUTZ UND ZUR NEUTRALISATION EINER NADEL ZUM
MEDIZINISCHEN GEBRAUCH

DEVICE FOR PROTECTING AND NEUTRALISING A NEEDLE FOR MEDICAL USE

(84) Etats contractants désignés:
**AT BE CH CY DE DK ES FI FR GB GR IE IT LI LU
MC NL PT SE**

(30) Priorité: **15.05.1998 FR 9806277
30.12.1998 FR 9816701
04.02.1999 FR 9901284**

(43) Date de publication de la demande:
**21.02.2001 Bulletin 2001/08**

(73) Titulaire: **Device Research & Development (DRD)
1235 Luxembourg (LU)**

(72) Inventeur: **JEREMIJEVIC, Vojin
F-93100 Montreuil-sous-Bois (FR)**

(74) Mandataire: **Flavenot, Bernard
Société ABRITT
17, rue du Docteur Charcot,
La Norville
91290 Arpajon (FR)**

(56) Documents cités:
EP-A- 0 321 903          EP-A- 0 344 606
EP-A- 0 713 710          EP-A- 0 819 441
US-A- 4 911 706          US-A- 5 295 972
US-A- 5 487 733          US-A- 5 713 872
US-A- 5 743 888

## Description

La présente invention concerne les dispositifs de protection et de neutralisation des aiguilles comportant une extrémité pointue et une extrémité de base, qui trouvent une application particulièrement avantageuse dans la protection et la neutralisation, notamment, des aiguilles de seringues, et plus particulièrement les dispositifs de protection et de neutralisation des aiguilles destinées à être utilisées par les professionnels du domaine médical ou analogue.

Il existe déjà des dispositifs de protection et de neutralisation d'une aiguille. Un tel dispositif est par exemple décrit dans le US-A-5 713 872. Le dispositif décrit dans ce document comporte essentiellement un manchon, une embase, une patte et une came reliant le manchon et l'embase. La configuration de ce dispositif dans son état initial est strictement identique celle qu'il a dans son état final, ce qui ne permet pas à ses utilisateurs, par exemple les infirmières, de savoir si l'aiguille de la seringue a été utilisée ou non. En outre, ce dispositif ne permet pas d'utiliser un système de neutralisation automatique de l'aiguille après, par exemple, sa première utilisation, obligeant ainsi l'utilisateur à effectuer une opération supplémentaire, en l'occurrence glisser un petit anneau autour de la patte, d'où une possibilité d'oublier cette opération avec toutes les conséquences pouvant être entraînées par un tel oubli.

Aussi, la présente invention a-t-elle pour but de réaliser un dispositif de protection et de neutralisation d'une aiguille à usage médical ou analogue, qui pallie en grande partie les inconvénients des dispositifs de l'art antérieur, qui présente une structure très simple avec laquelle il est possible d'obtenir une position origine, une position d'utilisation et une position de blocage qui soient visuellement différentes les unes des autres, et qui permette la neutralisation automatique de l'aiguille en passant de sa position d'utilisation à sa position de blocage, tout en permettant une protection de l'extrémité de l'aiguille, que le dispositif soit dans sa position d'origine ou dans sa position de blocage.

Plus précisément, la présente invention a pour objet un dispositif de protection et de neutralisation d'une aiguille à usage médical ou analogue comme défini dans la revendication 1.

D'autres caractéristiques et avantages de la présente invention apparaîtront au cours de la description suivante donnée en regard des dessins annexés à titre illustratif, mais nullement limitatif dans lesquels :

La figure 1 représente le schéma de principe du dispositif selon l'invention, de protection et de neutralisation d'une aiguille à usage médical ou analogue,
La figure 2 représente une vue en perspective d'un mode de réalisation préférentiel du dispositif selon l'invention,
Les figures 3 à 5 représentent une vue en coupe partielle longitudinale du mode de réalisation illustré sur la figure 2, respectivement dans trois positions dans lesquelles peut se trouver le dispositif selon l'invention lors de son utilisation, qui seront respectivement dénommées ci-après dans la description par "position d'origine" (fig 2 et 3), "position d'utilisation" (fig 4) et "position de blocage" (fig 5),
La figure 6 représente une vue schématique d'un autre mode de réalisation du dispositif selon l'invention,
Les figures 7 et 8 représentent deux vues en coupe schématique orthogonales d'un autre mode de réalisation du dispositif selon l'invention, la figure 7 étant une vue en coupe référencée I-I sur la figure 8 et la figure 8 étant une vue en coupe référencée II-II sur la figure 7,
Les figures 9 et 10 représentent une vue partielle du dispositif selon les figures 7 et 8, respectivement dans deux configurations, la figure 9 représentant une partie du dispositif dans une configuration dite "intermédiaire" correspondant à une préparation de la neutralisation de l'aiguille, la figure 10 représentant la même partie du dispositif dans la configuration où l'aiguille est complètement neutralisée, et
Les figures 11 et 12 représentent deux modes de réalisation des moyens d'embase illustrés schématiquement sur les figures 1 à 10.

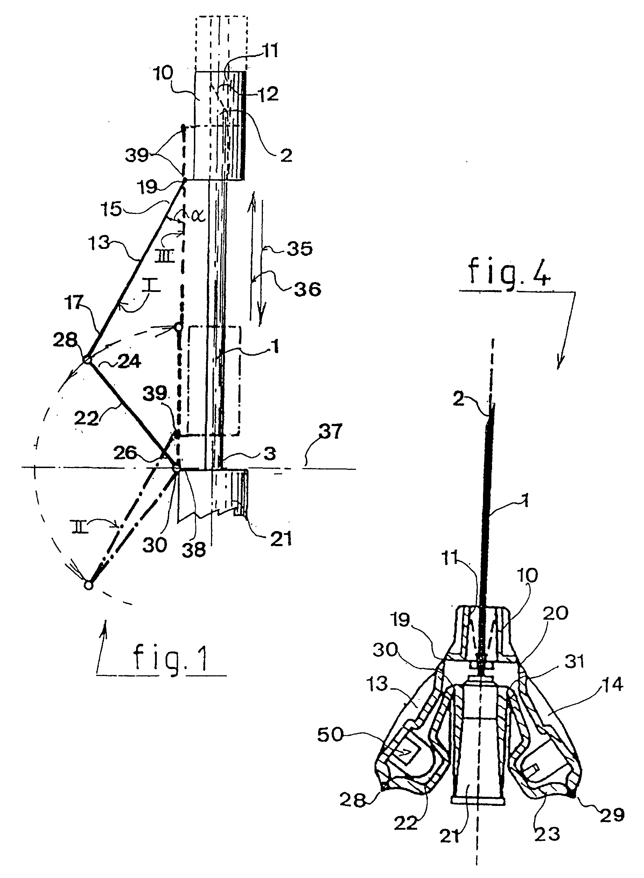

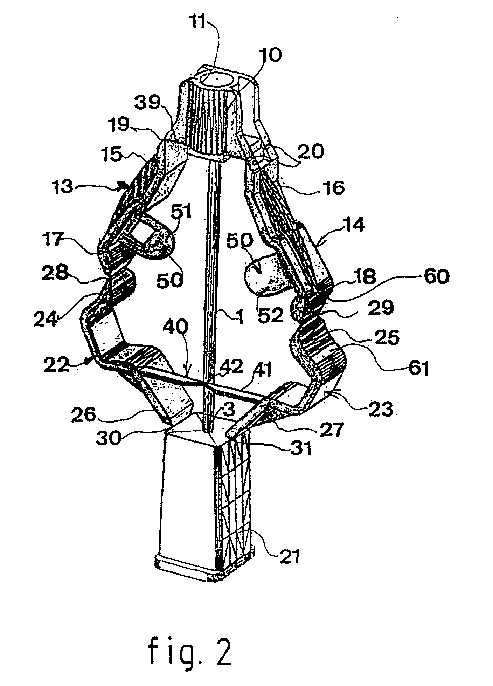

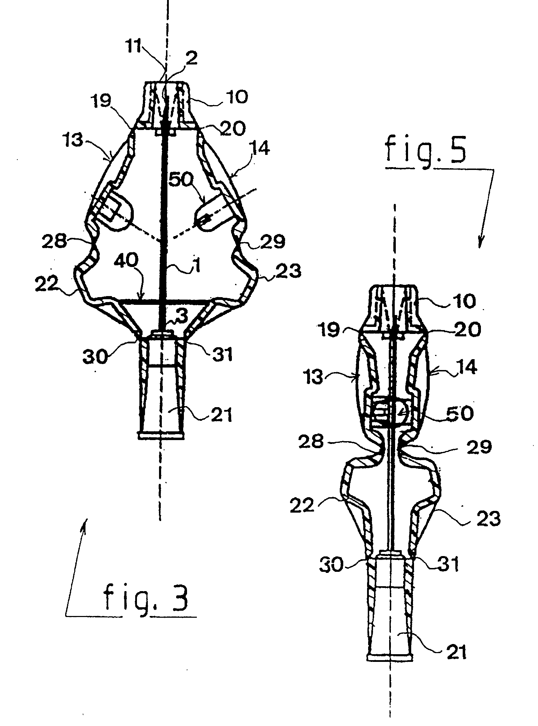

La figure 1 représente le schéma de principe d'un dispositif selon l'invention, de protection et de neutralisation d'une aiguille 1 à usage médical ou analogue comprenant une extrémité pointue 2 et une extrémité de base 3, sachant que ce schéma de principe peut également être une représentation schématique d'un mode de réalisation du dispositif.

Par référence à ce schéma, le dispositif comporte un manchon 10 comportant une percée traversante 11 définie suivant un axe donné 12, cette percée traversante 11 ayant une section au moins égale à celle de l'aiguille à protéger de façon que le manchon puisse facilement coulisser avantageusement sur toute la longueur de l'aiguille 1.

Dans cette réalisation, le dispositif comporte une patte 13 comportant une première 15 et une seconde 17 extrémités, cette patte ayant une longueur "$L_1$" définie entre ses deux extrémités.

Cette patte 13 est associée à un point 39 du manchon 10 par des premiers moyens de charnière à rappel élastique 19 qui relient la première extrémité 15 de la patte 13 avec le manchon 10, ces premiers moyens de charnière étant montés avec la patte de façon que cette dernière prenne une position d'équilibre définie sur une direction faisant avec l'axe 12 de la percée traversante un angle aigu a pour que, lorsqu'elle est écartée de cette position d'équilibre sous l'action d'une force quelconque, elle reprenne automatiquement cette position dès que l'action de la force est annulée.

Le dispositif comporte en outre des moyens d'embase 21 apte à recevoir l'extrémité de base 3 de l'aiguille 1. Ces moyens 21 peuvent être de tous types. Ils peuvent par exemple être constitués d'un embout à emmanchement conique, mais il est bien évident qu'ils peuvent prendre toutes autres formes en fonction de la nature et de l'utilisation de l'aiguille. Ces moyens peuvent même être constitués par une partie d'une seringue ou analogue.

Ces moyens 21 peuvent aussi être constitués par une gaine ou analogue apte à venir entourer, en l'emboîtant, la partie base 3 de l'aiguille à protéger 1. Ce dernier mode de réalisation est celui qui est représenté sur les figures 1 à 5.

Le dispositif selon la figure 1 comporte aussi une came de liaison 22 définie entre une première 24 et une seconde 26 extrémités. Cette came de liaison 22 a une longueur "$l_1$" définie entre ses deux extrémités 24 et 26, la longueur "$l_1$" étant au plus égale à la longueur "$L_1$" de la première patte.

De façon schématique comme illustré sur la figure 1, le dispositif comporte enfin des premiers moyens 28 pour monter chacune des première 24 et seconde extrémités 26 de la came de liaison 22 en rotation libre respectivement sur la seconde extrémité 17 de la patte 13 et, en point 38, sur les moyens d'embase 21.

Le dispositif tel que décrit ci-dessus fonctionne et s'utilise de la façon suivante:

Il est tout d'abord supposé que le dispositif se trouve dans la configuration (I) dite "position d'origine" qui est représentée sur la figure 1 en traits pleins et sur les figures 2 et 3. Dans cette positon, l'extrémité pointue 2 de l'aiguille est située à l'intérieur de la percée traversante 11 réalisée dans le manchon 10 et s'y trouve ainsi protégée.

Dans la pratique, le dispositif peut être associé à une aiguille 1 alors qu'il est dans cette configuration (I) puis, de façon connue, l'ensemble ainsi obtenu est stérilisé et commercialisé, par exemple dans une pochette stérile ou sous blister.

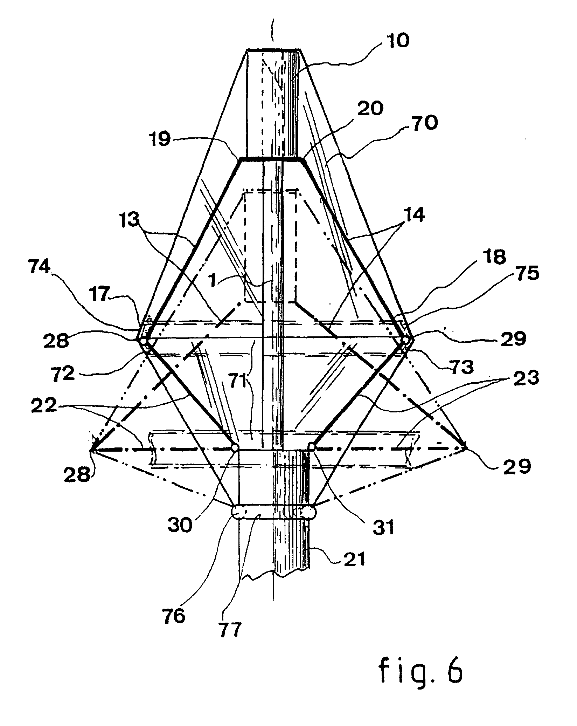

Quand l'aiguille doit être utilisée par le personnel soignant, l'ensemble "dispositif de protection - aiguille" est sorti de la pochette, puis le dispositif est amené dans la configuration (II) dite "position d'utilisation" représentée en traits-points sur la figure 1 et sur la figure 4.

Pour amener le dispositif de protection de l'aiguille, de la position d'origine (I) à la position d'utilisation (II), l'utilisateur exerce, par exemple manuellement, un couple de forces respectivement sur le manchon 10 et sur l'embase 21, pour rapprocher le manchon de l'embase dans un glissement représenté par la flèche 35.

Dans ce mouvement, la charnière élastique 19 permet à la patte 13 de subir tout d'abord une première rotation dextrorsum, selon la représentation sur la figure 1, autour du point de charnière 39. Ce mouvement est imposé par le déplacement en rotation senestrorsum de la came 22 autour du point de rotation libre 30. Quand le point de rotation libre 28 dépasse et vient en dessous du plan 37 perpendiculaire à l'axe 12 et passant par le point 38 de l'embase défini ci-avant, par l'effet de ressort de rappel de la charnière 19 tel que défini ci-avant, la patte 13 a alors tendance à subir une rotation inverse de la précédente, c'est-à-dire dans le sens senestrorsum selon la représentation sur la figure 1, qui la ramène vers sa position origine par rapport à l'axe 12.

Sous l'action de cette force de rappel élastique qui peut s'ajouter au couple de forces exercé entre le manchon 10 et l'embase 21, la patte 13 entraîne encore plus rapidement le manchon vers l'embase et le dispositif prend la position d'utilisation (II) illustrée en traits-points sur la figure 1.

Dans cette configuration (II), l'aiguille 1 est complètement dégagée. Elle peut alors être utilisée de façon classique pour la fonction pour laquelle elle est prévue, sachant que cette fonction n'a pas besoin d'être décrite ici puisque l'utilisation d'une aiguille est bien connue en elle-même.

Quand l'aiguille a été utilisée, l'utilisateur peut repousser le manchon 10 vers l'extrémité pointue 2 de l'aiguille pour tendre à lui faire reprendre sa position d'origine, le manchon 10 coulissant le long de l'aiguille 1 selon la flèche 36 jusqu'à ce que l'extrémité pointue 2 de l'aiguille soit située dans la percée 11 et soit recouverte par le manchon 10.

Le passage du dispositif de sa position d'utilisation (II) à sa position d'origine (I) peut être effectué manuellement en exerçant une force de poussée sur l'extrémité 24 de la came 22 par rapport à l'embase 21.

Il est rappelé que la figure 1 représente un schéma de principe du dispositif selon l'invention, ainsi qu'un premier mode de réalisation de ce dispositif. Dans ce mode de réalisation, le dispositif ne comporte qu'une seule patte 13 et qu'une seule came de liaison 22 et, bien qu'il fonctionne parfaitement, il est cependant avantageux de lui donner une structure comme celle qui est illustrée sur les figures 2 à 5.

Le mode de réalisation du dispositif illustré sur les figures 2 à 5 est avantageux car sa structure est symétrique et donc équilibrée, ce qui améliore son fonctionnement par rapport à celui selon la réalisation illustrée sur la figure 1.

Dans le mode de réalisation illustré sur les figures 2 à 5, le dispositif selon l'invention comporte en plus, par rapport à la réalisation illustrée sur la figure 1, une seconde patte 14 définie entre une première 16 et une seconde 18 extrémités, cette seconde patte ayant une longueur "$L_2$" définie entre ses deux extrémités, des seconds moyens de charnière à rappel élastique 20 pour relier la première extrémité 16 de la seconde patte avec le manchon 10, ces seconds moyens de charnière étant agencés pour que la seconde patte prenne une position d'équilibre définie sur une direction faisant avec l'axe de la percée traversante un angle aigu a', une seconde came de liaison 23 définie entre une première 25 et une seconde 27 extrémités, la seconde came de liaison ayant une longueur "$l_2$" définie entre ses deux

extrémités, la longueur "l$_2$" étant au plus égale à la longueur "L$_2$" de la patte, et des seconds moyens 29, 31 pour monter chacune des première et seconde extrémités de la seconde came de liaison en rotation libre respectivement sur la seconde extrémité de la seconde patte 14 et sur les moyens d'embase 21.

Dans le mode de réalisation avantageux illustré, dans le but d'obtenir une parfaite symétrie du dispositif par rapport à l'axe 12, les longueurs "L$_1$" et "L$_2$" sont sensiblement égales à une même valeur "L" et les longueurs "l$_1$" et "l$_2$" sont sensiblement égales à une même valeur "l".

En outre, pour faciliter la réalisation industrielle du dispositif et minimiser son encombrement, il est avantageux que les deux pattes 13, 14 et les deux cames de liaison 22, 23 soient sensiblement situées dans un même plan et forment sensiblement un quadrilatère dont les diagonales sont sensiblement perpendiculaires, la diagonale reliant les sommets du quadrilatère respectivement situés sur le manchon 10 et sur les moyens d'embase 21 étant sensiblement confondue avec l'axe 12 de la percée traversante 11.

Il est aussi possible qu'au moins deux des éléments suivants: manchon 10, moyens d'embase 21, patte 13, 14, came 22, 23, moyens de charnière 19, 20, moyens de montage en rotation libre 28, 29, 30, 31, soient réalisés dans un même matériau, par exemple de la matière plastique.

Cependant, il est particulièrement avantageux de réaliser tous les éléments constitutifs du dispositif dans ce même matériau, ce qui permet de le réaliser d'une seule pièce par la méthode bien connue du moulage. Sur ce dernier point, les figures 2 à 5 montrent, à titre d'exemple, la forme qui peut être donnée à chacun des éléments réalisés dans une matière plastique par la technique du moulage, ainsi que les moyens de renfort pour rigidifier les pattes et les cames.

Ce procédé de fabrication permet aussi de réaliser les moyens de charnières 19, 20 et les moyens de montage en rotation libre 28, 29, 30, 31, directement en même temps que les autres éléments constitutifs du dispositif. Notamment, la fonction de ces moyens de charnières à rappel élastique et de ces moyens de montage en rotation libre est facilement obtenue en donnant une forme déterminée à la partie de matière plastique qui relie, d'une part les pattes 13, 14 au manchon, et d'autre part les cames de liaison 22, 23 avec les pattes et les moyens d'embase 21.

De telles réalisations de charnières à rappel élastique et de charnières en rotation libre qui utilisent les caractéristiques de la matière et des formes particulières comme des amincissements de parois ou analogues, sont bien connues des plasticiens et ne seront pas plus amplement décrites ici.

Pour rigidifier l'ensemble du dispositif quand il est dans sa position d'origine avant sa première utilisation, il est avantageux qu'il comporte en outre des moyens de lien 40 relativement rigides reliant directement ou indirectement une patte 13 et une came de liaison 22.

Ces moyens de lien 40 ont été représentés sur les figures 2 et 3 dans le cas où le dispositif a une structure en forme de quadrilatère. Dans ce mode de réalisation, les moyens de lien 40 sont constitués par une barrette 41 ou analogue reliée, par ses deux extrémités, aux deux cames 22, 24 et comportant, par exemple en son milieu, un point de faiblesse 42. Dans ce cas, la liaison entre la patte 13 et la came 22 est une liaison indirecte.

Cette barrette 41 peut cependant relier une patte et une came selon d'autre configuration que celle qui est illustrée. L'homme du métier choisira la configuration qui conviendra le mieux en fonction de l'utilisation du dispositif et/ou de son procédé de fabrication. Par exemple, il pourra choisir une réalisation de barrette 41 qui relie l'extrémité 15 de la patte 13 et l'extrémité 26 de la came 22, ou bien une barrette reliant le manchon 10 et l'embase 21.

De façon connue, le point de faiblesse 42 permet d'obtenir, sous l'effet d'une force donnée, une rupture, à son niveau, des moyens de lien tels que la barrette 41. Cette force peut être obtenue, par exemple en exerçant directement un effort sur les deux parties de barrette situées de part et d'autre du point de faiblesse 41, ou indirectement en faisant passer le dispositif de sa position d'origine (I), figure 3, à sa position d'utilisation (II), figure 4.

Cette caractéristique est avantageuse car elle permet, comme mentionné ci-avant, de rigidifier le dispositif quand il est dans sa position d'origine (I), mais aussi parce qu'elle permet d'indiquer à tout utilisateur d'une aiguille munie d'un tel dispositif de protection s'il y a un risque pour qu'elle ait déjà été utilisée. En effet, une barrette entière est un gage certain d'une non-utilisation de l'aiguille, tandis qu'une barrette cassée est le signe d'une éventuelle utilisation antérieure, auquel cas, par sécurité, il sera plus prudent de ne pas l'utiliser.

Comme il a été mentionné précédemment, quand l'aiguille a été utilisée, il est possible de ramener le dispositif dans sa position d'origine (I), figure 3, ce qui permet au moins de cacher l'extrémité pointue de l'aiguille. Cependant, avec les seuls éléments de la structure décrite ci-dessus, il y a un risque pour que cette extrémité pointue se trouve, par inadvertance, à nouveau dégagée de l'intérieur du manchon et pour que l'aiguille, par exemple, soit à nouveau utilisée.

Aussi, est-il avantageux que le dispositif selon l'invention comporte en outre des moyens encliquetables 50 pour bloquer, de façon directe ou indirecte, la patte et la came de liaison l'une par rapport à l'autre dans une position donnée, dans laquelle l'extrémité pointue de l'aiguille ne peut plus sortir de l'intérieur du manchon. Cette position est par exemple celle qui permet au dispositif de prendre la position (III) dite "position de blocage" illustrée sur la figure 5 et schématisée en traits interrompus sur la figure 1. Dans cette position de blocage (III) du dispositif, l'extrémité pointue de l'aiguille est définitivement maintenue dans le manchon, ce qui empêche tout risque de blessure ou de réutilisation.

Les figures 2 à 5 représentent un mode de réalisation de ces moyens encliquetables 50 pour bloquer la patte et la came de liaison l'une par rapport à l'autre dans une position donnée. Ces moyens de blocage 50 sont constitués par deux crochets 51, 52 solidaires respectivement des deux pattes 13 et 14, et qui s'accrochent l'un dans l'autre lorsque, sous l'action d'une pression exercée sur les deux pattes, ces dernières sont amenées très proches l'une de l'autre, comme représenté sur la figure 5.

La longueur du manchon 10, celle des pattes et celle des cames sont déterminées de façon que, lorsque le dispositif de protection est dans cette position de blocage (III), les pattes et les cames soient sensiblement alignées et que l'extrémité pointue 2 de l'aiguille 1 soit située dans le manchon 10.

Pour obtenir ce résultat, il est nécessaire que la longueur "M" du manchon, la longueur "L" de la patte 13 (ou des pattes 13, 14) et la longueur "l" de la came 22 (ou des cames 22, 23) soient déterminées de façon que les sommes L + l et L + l + M encadrent la longueur "A" de l'aiguille 1 à protéger mesurée entre son extrémité pointue 2 et son extrémité de base 3, c'est-à-dire de façon que ces longueurs vérifient la relation suivante :

$$L + l < A < L + l + M$$

Il a été décrit ci-dessus un exemple de réalisation des moyens de blocage 50, mais il est bien évident qu'il peut en exister d'autres types, par exemple des cliquets ou analogues positionnés sur les deux portions recourbées 60, 61 qui bordent les moyens de montage en rotation libre 28, 29 entre respectivement les pattes 13, 15 et les cames 22, 23 et qui viennent en regard l'une de l'autre quand le dispositif est dans sa position de blocage (III). Ces cliquets n'ont pas été représentés, dans l'unique souci d'une simplification des dessins. Ils seront par exemple constitués par deux ergots situés respectivement sur les deux portions recourbées 60, 61 et comportant, par exemple, des embouts courbes conformés pour passer l'un sur l'autre lorsque les pattes et les cames sont amenées en position d'alignement, et pour ne plus pouvoir se désunir dans un mouvement en sens contraire.

Ces moyens de blocage encliquetables peuvent aussi être constitués par deux éléments à emboîtement mâle-femelle inviolable, comportant, respectivement sur leur paroi externe et sur leur paroi interne, des dentures orientées complémentaires qui ne peuvent se déplacer les unes par rapport aux autres que dans un sens de déplacement de l'élément mâle dans l'élément femelle. Ce type de dentures est bien connu en lui-même par les hommes du métier et ne sera donc pas plus amplement décrit ici.

La figure 6 représente un schéma d'un autre mode de réalisation du dispositif selon l'invention.

Comme illustré sur la figure 6, le dispositif selon l'invention, de protection et de neutralisation d'une aiguille 1 à usage médical ou analogue, comporte, en plus des caractéristiques essentielles décrites ci-avant en regard des figures 1 à 5, une enveloppe 70 en un matériau élastique enveloppant sous tension l'ensemble constitué par le manchon 10, les première et seconde pattes 13, 14, les premiers et seconds moyens de charnière 19, 20, les première et seconde cames de liaison 22, 23, les premiers et seconds moyens 28, 29, 30, 31 pour monter chacune des première et seconde extrémités des première et seconde cames de liaison respectivement sur la seconde extrémité 17, 18 des première et seconde pattes 13, 14 et sur les moyens d'embase 21, et au moins une partie des moyens d'embase 21.

Le matériau élastique dans lequel est réalisée l'enveloppe 70 est transparent, ou sensiblement transparent, de façon que les utilisateurs du dispositif puissent voir à travers la paroi de l'enveloppe et donc voir notamment l'aiguille et toutes les indications que peuvent éventuellement porter les différents éléments mentionnés ci-avant.

L'aiguille 1 est ainsi protégée de l'environnement extérieur, comme avec une enveloppe connue sous le terme de "blister". Mais, contrairement au blister de l'art antérieur qu'il est nécessaire d'enlever avant usage, il n'est pas besoin d'enlever l'enveloppe 70 avant d'utiliser l'aiguille. En effet, quand l'aiguille sort du manchon comme explicité ci-avant en regard des figures 1 à 5, elle traverse sans difficulté la paroi de l'enveloppe 70 et, comme de plus le matériau dans lequel est réalisée l'enveloppe est élastique, cette paroi se resserre autour de l'aiguille pour constituer un passage étanche.

Le dispositif peut comporter en outre un sachet 71 ou analogue disposé à l'intérieur de l'enveloppe 70, réalisé en un matériau non extensible et contenant un produit donné.

Ce sachet est en outre apte à se déchirer sous une traction donnée et des moyens 72, 73 sont prévus pour solidariser deux de ses points 74, 75 avec deux points sensiblement opposés de la paroi intérieure de l'enveloppe 70 avantageusement situés sensiblement en regard des premiers et seconds moyens 28, 29 pour monter la première extrémité des première et seconde cames de liaison en rotation libre sur la seconde extrémité des première et seconde pattes.

A titre d'exemple, le produit contenu dans le sachet 71 présente au moins l'une des propriétés suivantes: être apte à absorber au moins une partie de la lumière visible, être apte à se durcir, être apte à stériliser.

De cette façon, quand l'aiguille 1 est utilisée une première fois comme explicité ci-avant, et que les deux charnières 28, 29 s'éloignent l'une de l'autre quand le manchon est descendu le long de l'aiguille, les deux points 74, 75 s'éloignent aussi l'un de l'autre, ce qui a pour effet de tirer sur le sachet qui se déchire. Le produit qu'il contient se répand alors dans l'enveloppe 70.

Cependant, la déchirure du sachet 71 ne se produit que lorsque l'extrémité pointue 2 de l'aiguille 1 a déjà traversé la paroi de l'enveloppe et est prête à être utilisée pour une injection.

Il est à noter que le produit répandu dans l'enveloppe ne peut pas sortir de celle-ci car, comme mentionné ci-avant, la paroi de l'enveloppe perforée par l'aiguille se resserre autour de l'aiguille et constitue un passage étanche.

Ainsi, dans le cas où le produit est apte à absorber au moins une partie de la lumière visible, c'est-à-dire dans le cas où il est coloré, il sera visible pour l'oeil humain et, si ce produit coloré a empli l'enceinte de l'enveloppe, les utilisateurs du dispositif seront informés du fait que l'aiguille a potentiellement déjà été utilisée.

Dans le cas où le produit est apte à se durcir, lorsque l'aiguille aura été utilisée et que l'ensemble du dispositif a été remis dans sa configuration d'origine après l'injection, les pattes, cames, manchon et embase seront englobés dans le produit et solidarisés les uns aux autres. Il ne sera donc plus possible de réutiliser l'aiguille.

Enfin, dans le cas où le produit est apte à stériliser des objets, lorsque le dispositif est ramené dans sa position d'origine après utilisation de l'aiguille, cette dernière baignera dans le produit répandu dans l'enveloppe et sera donc stérilisée par ce produit, ce qui pourra éviter une contamination si une personne venait à se piquer avec l'extrémité pointue de l'aiguille qui serait par inadvertance ressortie de l'enveloppe.

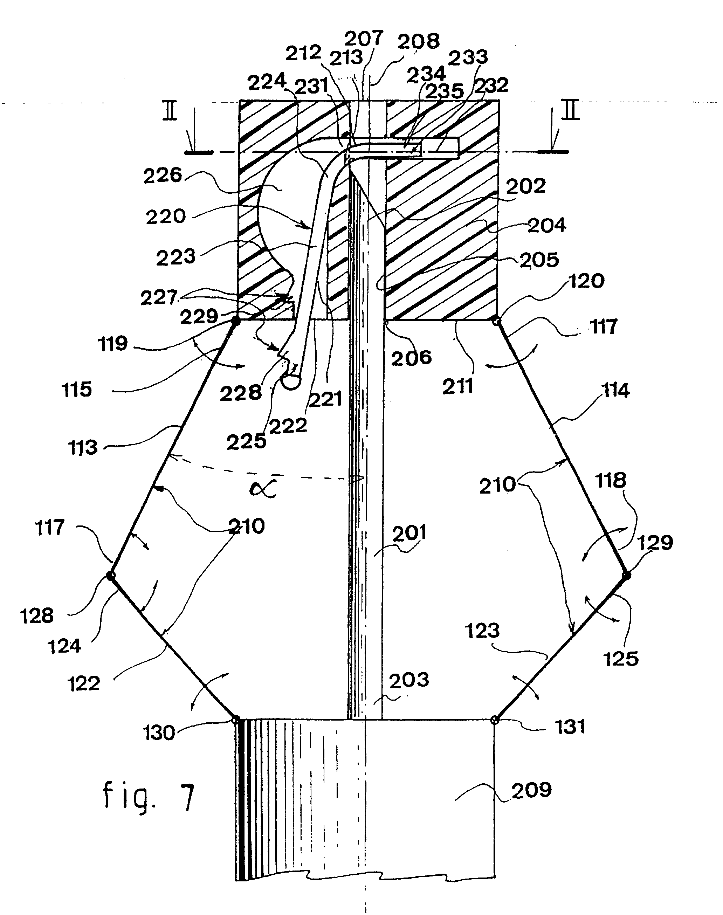

Les figures 7 à 10 représentent un autre mode de réalisation d'un dispositif selon l'invention, de protection et de neutralisation d'une aiguille à usage médical ou analogue.

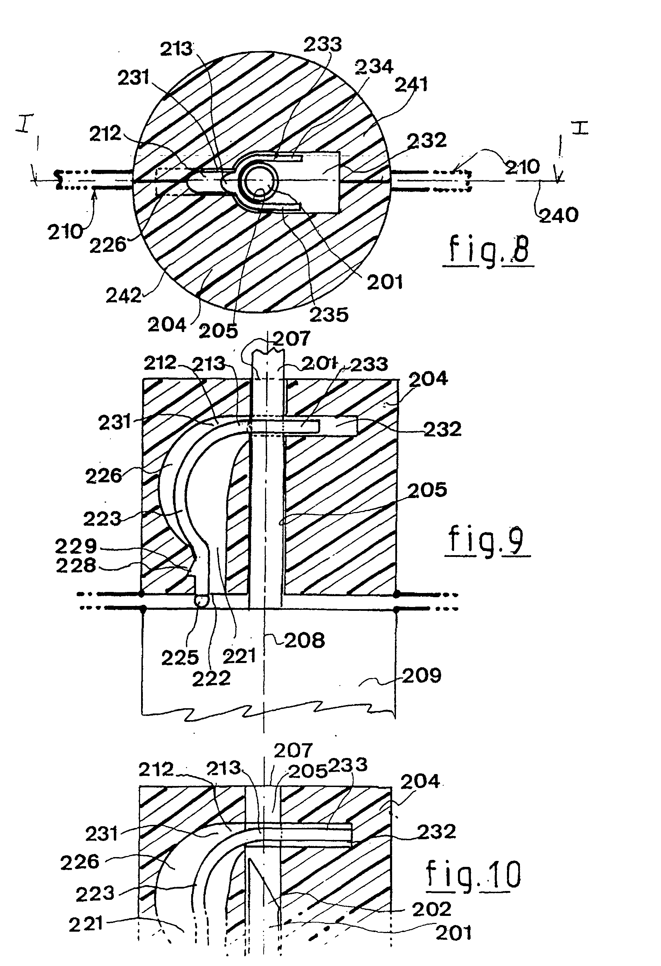

Ce dispositif selon les figures 7 et 8 comprend un manchon 204 comprenant une percée traversante 205, cette percée traversante comportant deux première 206 et seconde 207 sorties, cette percée traversante étant définie suivant un axe donné 208 et avant une section transversale au moins égale à celle de l'aiguille 1 de façon que l'aiguille soit apte à coulisser dans cette percée traversante.

Il comporte en outre une embase 209 solidaire de l'extrémité de base 203 de l'aiguille 201, cette embase permettant de façon connue de réunir l'aiguille à une seringue, et des moyens de lien élastique 210 reliant le manchon 204 et l'embase 209, ces moyens de lien élastique permettant au manchon 204 de coulisser le long de l'aiguille 201, celle-ci pénétrant dans la percée traversante 205 du manchon par sa première sortie 206, de façon que le manchon soit apte à prendre deux positions extrêmes, une première position dans laquelle il entoure l'extrémité pointue 202 de l'aiguille, cette extrémité pointue étant située à une distance donnée de la seconde sortie 207 de la percée traversante 205, et une seconde position dans laquelle la face 211 du manchon 204 comportant la première sortie 6 de la percée traversante est positionnée à proximité de l'embase 209.

Ces moyens de liens élastiques 210 sont par exemple constitués par les deux pattes 113, 114 comportant respectivement une première 115, 116 et une seconde 117, 118 extrémités, ces pattes ayant une longueur "L" définie entre leurs deux extrémités, des premiers moyens de charnière à rappel élastique 119, 120 pour relier la première extrémité 115, 116 des deux pattes avec le manchon 4, ces premiers moyens de charnière étant agencés pour que les deux pattes prennent une position d'équilibre définie sur une direction faisant avec l'axe de la percée traversante un angle aigu a, deux cames de liaison 122, 123, ces cames de liaison étant respectivement définies entre une première 124, 125 et une seconde 126, 127 extrémités et ayant une longueur "l" définie entre leurs deux extrémités, la longueur "1" des cames de liaison étant au plus égale à la longueur "L" des pattes, et des premiers moyens 128, 129, 130, 131 pour monter chacune des première et seconde extrémités respectivement des deux cames de liaison en rotation libre respectivement sur la seconde extrémité 117, 118 des deux pattes et sur l'embase 221. De façon avantageuse, les deux pattes et les deux cames de liaison sont sensiblement situées dans un même plan et forment sensiblement un quadrilatère dont les diagonales sont sensiblement perpendiculaires. En outre il est avantageux que la longueur "L" des pattes et celle "l" des cames de liaison soient déterminées de façon que la somme L + l et la somme L + l + M, "M" représentant la longueur du manchon 204, encadre la longueur de l'aiguille 1 à protéger, mesurée entre son extrémité pointue 202 et son extrémité de base 203.

Le dispositif comporte en outre une première portion de canal 212 réalisée dans le manchon 204 et coupant la percée traversante 205 dans sa partie comprise entre sa seconde sortie 207 et l'extrémité pointue 202 de l'aiguille 201 lorsque le manchon 204 est dans sa première position, un obturateur 213 monté coulissant dans cette première portion de canal 212, cet obturateur étant apte à prendre deux première et seconde positions, une première position dans laquelle il n'est pas situé dans la percée traversante 205 et une seconde position dans laquelle il est situé dans la percée traversante, et des moyens 220 pour appliquer une force pressante sur l'obturateur 213 quand le manchon 204 arrive à proximité de l'embase 209 en passant de sa première à sa seconde position.

Dans une réalisation préférentielle, ces moyens 220 comportent une seconde portion de canal 221 réalisée dans le manchon 204 en continuité de la première portion de canal 212 et débouchant par un orifice de sortie 222 sur la face 211 du manchon comportant la première sortie 206 de la percée traversante 205, et une tige flexible 223 préformée en arc et montée coulissante dans cette seconde portion de canal 221. Une première 224 des deux extrémités de la tige flexible est liée à l'obturateur 213 et son autre extrémité 225 émerge initialement de l'orifice de sortie 222 de la seconde portion de canal d'une quantité au moins égale à la distance que doit parcourir l'obturateur 213 pour passer de sa première à sa seconde position.

Ces moyens 220 comporte en outre une cavité de dégagement 226 bordant la seconde portion de canal 221 et en communication avec cette seconde portion de canal, la cavité de dégagement étant en outre déterminée, quant à sa configuration, de façon que, lorsque l'obturateur 213 est maintenu dans sa première position, la tige flexible 223 puisse se déformer en flexion comme un arc bandé et pénétrer latéralement dans cette cavité de dégagement 226 quand la face 211 du manchon comportant la première sortie 206 de la percée traversante 205 vient à proximité de l'embase 209.

Le dispositif comporte en outre des moyens 227 pour bloquer en position la seconde extrémité 225 de la tige flexible 223 quand cette dernière est rentrée dans la seconde portion de canal 221. Ces moyens 227 sont constitués par au moins un ergot 228 solidaire de la tige flexible 223 et un logement 229 complémentaire de l'ergot 228 réalisé dans la paroi de la seconde portion de canal 221 de façon à s'accoupler avec l'ergot à la manière d'un emboîtement du type mâle-femelle.

Dans une réalisation avantageuse comme celle qui est illustrée sur les figures, la première portion de canal 212 comporte au moins deux première 231 et seconde 232 parties réalisées de part et d'autre de la percée traversante 205, la première partie 231 de première portion de canal étant en continuité de la seconde portion de canal 221, l'obturateur 213 étant entièrement contenu dans cette première partie 231 de première portion de canal 212 quand il est dans sa première position.

Le dispositif comporte aussi un étrier 233 solidaire de l'obturateur 213 et sensiblement en forme de "U", les deux branches 234, 235 de l'étrier étant écartées l'une de l'autre d'une distance au moins égale au diamètre de l'aiguille 201 et cet étrier 233 étant configuré de façon que, lorsque l'obturateur 13 est dans sa première position, l'espace 236 défini entre les deux branches 234, 235 soit situé dans l'axe 208 de la percée traversante 205 et que les deux branches 234, 235 plongent au moins partiellement dans la seconde partie 232 de première portion de canal.

Dans une réalisation avantageuse, la tige flexible 223, l'obturateur 213 et l'étrier 233 sont réalisés en une seule pièce, par exemple en matière plastique par moulage.

Avec la structure définie ci-dessus, il est avantageux que les axes des deux portions de canal 212, 221 soient contenus dans un même plan 240 qui contient lui-même l'axe 208 de la percée traversante 205. Dans ce cas, le manchon 204 peut être réalisé en deux demi-coquilles 241, 242 agencées pour être assemblées suivant le plan 240 contenant les deux portions de canal 212, 221, ce qui permet de le réaliser, par exemple en matière plastique, par la technique du moulage avec un moule qui est très simple à réaliser. Pour obtenir le dispositif, il suffit de placer la pièce unique "tige flexible-obturateur-étrier" dans les deux portions de canaux et de l'enfermer en rabattant les deux demi-coquilles l'une sur l'autre, puis de souder ces deux demi-coquilles par exemple par faisceau laser.

Le dispositif décrit ci-dessus en regard des figures 7 et 8 fonctionne et s'utilise de la façon suivante:

Il est tout d'abord précisé que l'aiguille est présentée dans le dispositif dans une configuration comme celle illustrée sur les figures 7 et 8.

Dans cette configuration, l'obturateur 213 est entièrement contenu dans la première partie 231 de la première portion de canal 212, l'étrier 233 permettant le passage de l'aiguille 201, et l'extrémité 225 de la tige flexible 223 émerge par l'orifice 222 de la seconde portion de canal 221, l'extrémité pointue 202 de l'aiguille étant bien protégée puisque entourée par le manchon 204.

Lorsqu'un utilisateur veut procéder à une piqûre avec une telle aiguille, il fait glisser le manchon 204 vers l'embase 202. Dans ce déplacement, les deux pattes 113, 114 subissent respectivement des rotations dextrorsum et senestrorsum et les deux cames 122, 123 des rotations respectivement senestrorsum et dextrorsum. L'aiguille sort de la percée traversante 205 par sa sortie 207 en passant entre les deux branches 234, 235 de l'étrier 233.

Lorsque le manchon 204 arrive à proximité de l'embase 209, l'extrémité 225 de la tige 223 vient buter contre cette embase. Comme l'aiguille est positionnée entre les deux branches de l'étrier, l'obturateur 213 est maintenu en position et, lorsque son extrémité 225 est repoussée dans la seconde portion de canal 221, la tige 223 se plie comme un arc en pénétrant dans la cavité de dégagement 226. Lorsque l'extrémité 225 est presque entièrement rentrée dans la seconde portion de canal 221, l'ergot 228 vient se bloquer dans le logement 229. A ce stade, le dispositif prend la configuration illustrée sur la figure 9.

Lorsque la piqûre est terminée, l'utilisateur fait à nouveau glisser le manchon 204 le long de l'aiguille jusqu'à ce qu'il reprenne sa position illustrée sur la figure 7. Puisque son extrémité 225 est maintenue en place par l'emboîtement ergot-logement, la tige bandée 223 exerce sur l'obturateur 213 une force pressante. Tant que l'aiguille se trouve entre les deux branches 234, 235 de l'étrier, l'obturateur reste dans sa position initiale (figure 9). En revanche, quand le manchon reprend sa position initiale par rapport à l'embase 209, l'extrémité pointue 202 se dégage de l'étrier 233 et, sous l'action de la force pressante exercée par la tige bandée 223, l'obturateur se translate dans la première portion de canal 212 pour venir obturer la percée traversante 205. La translation de l'obturateur est notamment guidée par les deux branches 234, 235 de l'étrier qui plongeaient déjà dans la seconde partie 232 de la première portion de canal 212. Le dispositif se trouve dans la configuration illustrée sur la figure 10.

Il est alors impossible à un utilisateur d'utiliser à nouveau l'aiguille 201 puisqu'il ne peut plus faire glisser le manchon vers l'embase, l'extrémité pointue de l'aiguille venant automatiquement buter contre l'obturateur 213 qui l'empêche de sortir de la percée traversante 205.

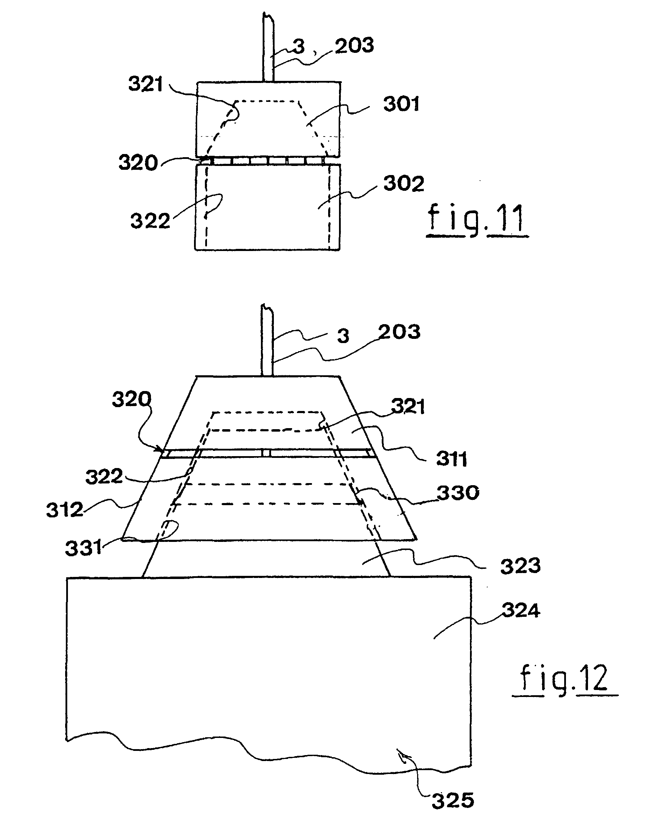

Les figures 11 et 12 représentent deux modes de réalisation particulièrement avantageux des moyens d'embase 21 ou 209 mentionnés ci-avant.

Dans ces deux modes de réalisation, les moyens d'embase 21 ou 209 comportent deux première et seconde bagues 301, 302 (figure 11), 311, 312 (figure 12), la première bague 301, 311 recevant l'extrémité basse 3, 203 de l'aiguille, et des moyens 320 pour relier les deux bagues entre elles par des points de faiblesse.

De plus, dans une réalisation possible, chacune des deux bagues comporte une percée 321, 322, les deux percées étant réalisées pour former, lorsque les deux bagues sont reliées entre elles, la partie femelle d'un emboîtement mâle-femelle, apte à coopérer avec la partie mâle complémentaire 323 constituée par un embout situé à l'extrémité du corps cylindrique 324 d'une seringue 325.

Dans une réalisation préférentielle, comme celle qui est illustrée sur la figure 12, la profondeur totale de ces deux percées lorsque les deux bagues sont reliées entre elles est inférieure à la hauteur de l'embout 323.

Il est aussi avantageux que les moyens d'embase comportent un anneau de non-retrait 330 situé sur la paroi 331 d'au moins l'une des deux percées.

Lorsque les moyens d'embase sont réalisés comme décrit en regard de la figure 11 ou de la figure 12, le dispositif selon l'invention apporte une encore plus grande sécurité d'utilisation des aiguilles à usage médical.

En effet, dans le cas du mode de réalisation des moyens d'embase selon la figure 11, il est avantageux de donner à la seconde bague 302 une longueur supérieure à la hauteur de l'embout 323, de façon à ne pas permettre l'emboîtement de cet embout avec la première bague 301. En conséquence, si le dispositif comporte cette seconde bague 302, il est certain que l'aiguille n'a pas été utilisée pour effectuer une injection. Si le dispositif ne comporte plus cette seconde bague 302, il est fort probable que l'aiguille a été utilisée et il est donc préférable de ne pas l'utiliser à nouveau.

Dans le cas du mode de réalisation des moyens d'embase selon la figure 12, l'embout 323 peut être enfiché en force dans les percées 321, 322 et, pour l'enlever, il faut exercer sur lui une traction relativement forte qui entraîne une rupture des points de faiblesse et une dissociation des deux bagues 311, 312, la bague 312 restant enfichée sur l'embout. L'accrochage de la seconde bague 312 sur l'embout 323 peut être renforcé par la présence de l'anneau de non-retrait 330 sur la paroi 331 de cette seconde bague.

## Revendications

**1.** Dispositif de protection et de neutralisation d'une aiguille (1, 201) à usage médical ou analogue comportant une extrémité pointue (2, 202) et une extrémité de base (3, 203), comprenant :

un manchon (10, 204) comportant une percée traversante (11, 206) définie suivant un axe (12, 208) donné, ladite percée traversante ayant une section au moins égale à celle de l'aiguille à protéger,
une première patte (13, 113, 14, 114) comportant une première (15, 115, 16, 116) et une seconde (17, 117, 18, 118) extrémités, cette patte ayant une longueur « L1 » définie entre ses deux extrémités,
des moyens d'embase (21, 209) aptes à recevoir l'extrémité de base (3, 203) de l'aiguille à protéger (1, 201),
une première came de liaison (22, 122, 23, 123), ladite première came de liaison étant définie entre une première (24, 124, 25, 125) et une seconde (26, 126, 27, 127) extrémités, cette came de liaison ayant une longueur « l1 » définie entre ses deux extrémités, la longueur « l1 » de la première came de liaison étant au plus égale à la longueur « L1 » de la première patte,
des premiers moyens (28, 128, 29, 129, 30, 130, 31, 131) pour monter chacune des première et seconde extrémités de la première came de liaison en rotation libre respectivement sur la seconde extrémité (17, 18) de la première patte et sur les moyens d'embase (21, 209), et des premiers moyens de chamière à rappel élastique (19, 20) pour relier la première extrémité (15, 16) de la première patte avec le manchon (10), et des moyens (50) pour bloquer ladite patte et ladite came de liaison l'une par rapport à l'autre dans une position de blocage (III) protégeant ainsi l'extrémité pointue de l'aiguille dans le manchon,

**caractérisé par le fait que** les premiers moyens de charnière (19,20) sont agencés pour que ladite première patte prenne une position initiale d'équilibre (I) définie sur une direction faisant avec l'axe de la percée traversante un angle aigu (a), de ce fait, le manchon (10) protège l'extrémité pointue (2, 202).

**2.** Dispositif selon la revendication 1, **caractérisé par le fait qu'**il comporte en outre des moyens de lien (40) reliant ladite patte (13, 14) et ladite came de liaison (22, 23) lorsqu'elles sont dans une première position donnée, ces moyens de lien comportant un point de faiblesse (41) permettant d'obtenir, sous l'effet d'une force donnée, une rupture des dits moyens de lien au niveau dudit point de faiblesse.

3. Dispositif selon l'une des revendications 1 et 2, **caractérisé par le fait que** les moyens (50) pour bloquer ladite patte et ladite came de liaison l'une par rapport à l'autre dans une seconde position donnée (fig.5) sont des moyens encliquetables.

4. Dispositif selon l'une des revendications 1 à 3, **caractérisé par le fait qu'**il comporte en outre :

une seconde patte (13, 14) comportant une première (15, 16) et une seconde (17, 18) extrémités, cette seconde patte ayant une longueur "$L_2$" définie entre ses deux extrémités, des seconds moyens de charnière à rappel élastique (19, 20) pour relier la première extrémité (15, 16) de la seconde patte avec le manchon (10), ces seconds moyens de charnière étant agencés pour que ladite seconde patte prenne une position d'équilibre définie sur une direction faisant avec l'axe de la percée traversante un angle aigu ($\alpha'$),
une seconde came de liaison (22, 23), ladite seconde came de liaison étant définie entre une première (24, 25) et une seconde (26, 27) extrémités, cette seconde came de liaison ayant une longueur "$l_2$" définie entre ses deux extrémités, la longueur "$l_2$" de la seconde came de liaison étant au plus égale à la longueur "$L_2$" de la seconde patte, et
des seconds moyens (28-31) pour monter chacune des première et seconde extrémités de la seconde came de liaison en rotation libre respectivement sur la seconde extrémité de la seconde patte (13, 14) et sur les moyens d'embase (21).

5. Dispositif selon la revendication 4, **caractérisé par le fait que** les longueurs "$L_1$" et "$L_2$" sont sensiblement égales à une même valeur "L" et que les longueurs "$l_1$" et "$l_2$" sont sensiblement égales à une même valeur "l".

6. Dispositif selon l'une des revendications 4 et 5, **caractérisé par le fait que** les deux première et seconde pattes et les deux première et seconde cames de liaison sont sensiblement situées dans un même plan et forment sensiblement un quadrilatère dont les diagonales sont sensiblement perpendiculaires, la diagonale reliant les sommets du quadrilatère respectivement situés sur le manchon (10) et sur les moyens d'embase (21) étant sensiblement confondue avec l'axe (12) de la percée traversante (11).

7. Dispositif selon l'une des revendications 1 à 6, **caractérisé par le fait qu'**au moins deux des éléments suivants : manchon (10), moyens d'embase (21), patte (13, 14), came de liaison (22, 23), moyens de charnière (19, 20), moyens de montage en rotation libre (28, 29, 30, 31), sont réalisés dans un même matériau.

8. Dispositif selon la revendication 7, **caractérisé par le fait qu'**au moins les deux dits éléments sont réalisés par moulage.

9. Dispositif selon la revendication 8, **caractérisé par le fait que** le matériau est une matière plastique.

10. Dispositif selon l'une des revendications 1 à 9, **caractérisé par le fait que** la longueur "$L_1$" de la patte et celle "$l_1$" de la came de liaison sont déterminées de façon que la somme $L_1 + l_1$ et la somme $L_1 + l_1 + M$, "M" représentant la longueur du manchon (10), encadrent la longueur "A" de l'aiguille (1) à protéger mesurée entre son extrémité pointue (2) et son extrémité de base (3).

11. Dispositif selon l'une des revendications 4 à 10, **caractérisé par le fait qu'**il comporte en outre une enveloppe (70) en un matériau élastique enveloppant sous tension l'ensemble constitué par le manchon (10), les première et seconde pattes (13, 14), les premiers et seconds moyens de charnière (19, 20), les première et seconde cames de liaison (22, 23), les premiers et seconds moyens (28-31) pour monter les premières et secondes extrémités des première et seconde cames de liaison en rotation libre respectivement sur la seconde extrémité (17, 18) des première et seconde pattes (13, 14) et sur les moyens d'embase (21), et au moins une partie des moyens d'embase (21).

12. Dispositif selon la revendication 11, **caractérisé par le fait que** le matériau élastique dans lequel est réalisée l'enveloppe (70) est transparent.

13. Dispositif selon l'une des revendications 11 et 12, **caractérisé par le fait qu'**il comporte en outre un sachet (71) réalisé en un matériau non extensible, le sachet (71) contenant un produit donné et étant apte à se déchirer sous une traction donnée, et des moyens (72, 73) pour solidariser le sachet (71) et l'enveloppe (70) sensiblement en deux points opposés (74, 75) de la paroi intérieure de l'enveloppe (70), les deux dits points se trouvant sensiblement en regard des premiers moyens (28, 29) pour monter la première extrémité des première et seconde cames de liaison en rotation libre sur la seconde extrémité des première et seconde pattes.

14. Dispositif selon la revendication 13, **caractérisé par le fait que** le produit contenu dans le sachet (71) présente au moins l'une des propriétés suivantes : être apte à absorber au moins une partie de la lumière visible, être apte à se durcir, être apte à stériliser.

15. Dispositif de protection et de neutralisation d'une aiguille (1, 201) à usage médical ou analogue selon l'une des revendications 1 à 14, ladite aiguille (201) comportant une extrémité pointue (2, 202) et une extrémité de base (203), comportant :

   un manchon (204) comprenant une percée traversante (205), cette percée traversante comportant deux première (206) et seconde (207) sorties, ladite percée traversante étant définie suivant un axe donné (208) et ayant une section transversale au moins égale à celle de l'aiguille (201), l'aiguille étant apte à coulisser dans ladite percée traversante,
   une embase (209) solidaire de l'extrémité de base (203) de l'aiguille (201), et
   des moyens de lien élastique (210) reliant le manchon (204) et l'embase (209), ces moyens de lien élastique permettant au manchon (204) de coulisser le long de l'aiguille (201), celle-ci pénétrant dans la percée traversante (205) du manchon par sa première sortie (206), ledit manchon étant apte à prendre deux positions extrêmes,

   * une première position dans laquelle le manchon entoure l'extrémité pointue (202) de l'aiguille, cette extrémité pointue étant située à une distance donnée de la seconde sortie (207) de la percée traversante (205),
   * une seconde position dans laquelle la face (211) du manchon (204) comportant la première sortie (206) de la percée traversante est positionnée à proximité de l'embase (209),

   **caractérisé par le fait qu'**il comporte en outre :

   une première portion de canal (212) réalisée dans le manchon (204) et coupant la percée traversante (205) dans sa partie comprise entre sa seconde sortie (207) et l'extrémité pointue (202) de l'aiguille (201) lorsque le manchon (204) est dans sa première position,
   un obturateur (213) monté coulissant dans la première portion de canal (212), ledit obturateur étant apte à prendre deux première et seconde positions, une première position dans laquelle il n'est pas situé dans la percée traversante (205), et une seconde position dans laquelle il est situé dans la percée traversante, et des moyens (220) pour appliquer une force pressante sur ledit obturateur (213) quand le manchon (204) arrive à proximité de l'embase (209) en passant de sa première à sa seconde position.

16. Dispositif selon la revendication 15, **caractérisé par le fait que** les moyens (220) pour appliquer une force pressante sur ledit obturateur quand le manchon arrive à proximité de l'embase en passant de sa première à sa seconde position sont constitués par :

   une seconde portion de canal (221) réalisée dans le manchon (204) en continuité de la première portion de canal (212) et débouchant par un orifice de sortie (222) sur la face (211) du manchon comportant la première sortie (206) de la percée traversante (205),
   une tige flexible (223) préformée en arc et montée coulissante dans cette seconde portion de canal (221) de façon qu'une première (224) de ses deux extrémités soit liée à l'obturateur (213) et que son autre seconde extrémité (225) émerge de l'orifice de sortie (222) de la seconde portion de canal d'une quantité au moins égale à la distance que doit parcourir l'obturateur (213) pour passer de sa première à sa seconde position,
   une cavité de dégagement (226) bordant la seconde portion de canal (221) et en communication avec cette seconde portion de canal, la cavité de dégagement étant déterminée de façon que, lorsque l'obturateur (213) est maintenu dans sa première position, la tige flexible (223) puisse se déformer en flexion et pénétrer latéralement dans cette cavité de dégagement (226) quand la face (211) du manchon comportant la première sortie (206) de la percée traversante (205) vient à proximité de l'embase (209).

17. Dispositif selon la revendication 16, **caractérisé par le fait qu'**il comporte en outre des moyens (227) pour bloquer en position la seconde extrémité (225) de la tige flexible (223) quand cette dernière est rentrée dans la seconde portion de canal (221).

18. Dispositif selon la revendication 17, **caractérisé par le fait que** les moyens (227) pour bloquer en position la seconde extrémité (225) de la tige flexible (223) quand cette dernière est rentrée dans la seconde portion de canal (221) sont constitués par au moins un ergot (228) solidaire de la tige flexible (223) et d'un logement (229) complémentaire de l'ergot (228) réalisé dans la paroi de la seconde portion de canal (221).

19. Dispositif selon l'une des revendications 15 à 18, **caractérisé par le fait que** la première portion de canal (212) comporte au moins deux première (231) et seconde (232) parties réalisées de part et d'autre de la percée traversante (205), la première partie (231) de la première portion de canal étant en continuité de la seconde portion de canal (221), l'obturateur (213) étant entièrement contenu dans cette première partie (231) de la première portion de canal (212) quand il est dans sa première position, et qu'il comporte en outre un étrier (233) solidaire de l'obturateur (213) et sensiblement en forme de "U", les deux branches (234, 235) de l'étrier étant écartées l'une de l'autre d'une distance au moins égale au diamètre de l'aiguille (201), cedit étrier (233) étant configuré de façon que, lorsque l'obturateur (213) est dans sa première position, l'espace (236) défini entre les deux branches (234, 235) soit situé dans l'axe (208) de la percée traversante (205) et que les deux branches (234, 235) plongent au moins partiellement dans la seconde partie (232) de la première portion de canal (212).

20. Dispositif selon la revendication 19, **caractérisé par le fait que** la tige flexible (223), l'obturateur (213) et l'étrier (233) sont réalisés en une seule pièce.

21. Dispositif selon la revendication 20, **caractérisé par le fait que** ladite pièce est réalisée en matière plastique par moulage.

22. Dispositif selon l'une des revendications 16 à 21, **caractérisé par le fait que** les axes des deux portions de canal (212, 221) sont contenus dans un plan (240) contenant l'axe (208) de la percée traversante (205), le manchon (204) étant réalisé en deux demi-coquilles (41, 42) agencées pour être assemblées suivant ledit plan (240) contenant les deux portions de canal (212, 221).

23. Dispositif selon l'une des revendications précédentes, **caractérisé par le fait que** les moyens d'embase (21, 209) comportent deux première et seconde bagues (301-302, 311-312), la première bague (301, 311) recevant l'extrémité basse (3, 203) de l'aiguille, et des moyens (320) pour relier les deux bagues entre elles par des points de faiblesse.

24. Dispositif selon la revendication 23, **caractérisé par le fait que** chacune des deux bagues comporte une percée (321, 322), les deux percées étant réalisées pour former, lorsque les deux bagues sont reliées entre elles, la partie femelle d'un emboîtement mâle-femelle apte à coopérer avec la partie mâle complémentaire constituée d'un embout (32) d'une seringue (325), la profondeur des deux percées lorsque les deux bagues sont reliées entre elles étant inférieure à la hauteur de l'embout de la seringue.

25. Dispositif selon l'une des revendications 23 et 24, **caractérisé par le fait qu'**il comporte en outre un anneau de non-retrait (330) situé sur la paroi (331) d'au moins l'une des deux percées,

## Patentansprüche

1. Vorrichtung zum Schützen und Neutralisieren einer Nadel (1, 201) für medizinischen Gebrauch oder dergleichen, wobei die Nadel ein spitzes Ende (2, 202) und ein Basisende (3, 203) besitzt, wobei die Vorrichtung umfaßt:

eine Hülse (10, 204) mit einer Durchgangsbohrung (11, 206), die auf einer gegebenen Achse (12, 208) definiert ist und einen Querschnitt besitzt, der nicht kleiner als jener der zu schützenden Nadel ist;
ein erstes Verbindungsglied (13, 113, 14, 114) mit einem ersten und einem zweiten Ende (15, 115, 16, 116, 17, 117, 18, 118), wobei das Verbindungsglied eine Länge "$L_1$" besitzt, die zwischen den beiden Enden definiert ist;
Basismittel (21, 209), die das Basisende (3, 203) der zu schützenden Nadel (1, 201) aufnehmen können;
einen ersten Kurbelarm (22, 122, 23, 123), der zwischen einem ersten und einem zweiten Ende (24, 124, 25, 125- 26, 126, 27, 127) definiert ist, wobei der Kurbelarm eine Länge "$l_1$" besitzt, die zwischen seinen beiden Enden definiert ist, wobei die Länge "$l_1$" des ersten Kurbelarms nicht größer als die Länge "$L_1$" des ersten Verbindungsgliedes ist;
erste Mittel (28, 128, 29, 129, 30, 130, 31, 131), die das erste und das zweite Ende des ersten Kurbelarms so anbringen, daß er am zweiten Ende (17, 18) des ersten Verbindungsgliedes bzw. an den Basismitteln (21, 209) frei schwenkbar ist, und
erste elastische Rückkehrschaniermittel (19, 20), die das erste Ende (15, 16) des ersten Verbindungsgliedes mit der Hülse (10) verbinden, und
Mittel (50), die das Verbindungsglied und den Kurbelarm relativ zueinander in einer Blockierposition (III) verriegeln, wodurch das spitze Ende der Nadel in der Hülse geschützt wird,

**dadurch gekennzeichnet, daß** die ersten Scharniermittel (19, 20) so beschaffen sind, daß das erste Verbindungsglied eine anfängliche definierte Gleichgewichtsposition (I) in einer Richtung einnimmt, die mit der Achse der Durchgangsbohrung einen spitzen Winkel (D) bildet, so daß die Hülse (10) das spitze Ende (2, 202) schützt.

2. Vorrichtung nach Anspruch 1, **dadurch gekennzeichnet, daß** sie ferner Stabmittel (40) enthält, die das Verbindungsglied (13, 14) mit dem Kurbelarm (22, 23) verbinden, wenn sie sich in einer ersten gegebenen Position befinden, wobei die Stabmittel einen Schwachpunkt (41) aufweisen, der bei Ausübung einer gegebenen Kraft ermöglicht, daß die Stabmittel an dem Schwachpunkt brechen.

3. Vorrichtung nach Anspruch 1 oder 2, **dadurch gekennzeichnet, daß** die Mittel (50) zum Verriegeln des Verbindungsgliedes und des Kurbelarms relativ zueinander in einer zweiten gegebenen Position (Fig. 5) Einrastbefestigungsmittel sind.

4. Vorrichtung nach einem der Ansprüche 1 bis 3, **dadurch gekennzeichnet, daß** sie ferner umfaßt:

ein zweites Verbindungsglied (13, 14) mit einem ersten Ende und einem zweiten Ende (15, 16 - 17, 18), wobei das zweite Verbindungsglied eine Länge "$L_2$" besitzt, die zwischen seinen beiden Enden definiert ist;
zweite elastische Rückkehrschamiermittel (19, 20), die das erste Ende (15, 16) des zweiten Verbindungsgliedes mit der Hülse (10) verbinden, wobei die zweiten Scharniermittel so beschaffen sind, daß das zweite Verbindungsglied eine definierte Gleichgewichtsposition in einer Richtung einnimmt, die mit der Achse der Durchgangsbohrung einen spitzen Winkel (□) bildet;
einen zweiten Kurbelarm (22, 23), der zwischen einem ersten und einem zweiten Ende (24, 25 - 26, 27) definiert ist und eine Länge "$l_2$" besitzt, die zwischen seinen beiden Enden definiert ist, wobei die Länge "$l_2$" des zweiten Kurbelarms nicht größer als die Länge "$L_2$" des zweiten Verbindungsgliedes ist; und
zweite Mittel (28, 31), die das erste und das zweite Ende des zweiten Kurbelarms so anbringen, daß er am zweiten Ende (13, 14) des zweiten Verbindungsgliedes bzw. an den Basismitteln (21) frei schwenkbar ist.

5. Vorrichtung nach Anspruch 4, **dadurch gekennzeichnet, daß** die Längen "$L_1$" und "$L_2$" im wesentlichen gleich einem gemeinsamen Wert "L" sind und daß die Längen "$l_1$" und "$l_2$" im wesentlichen gleich einem gemeinsamen Wert "l" sind.

6. Vorrichtung nach Anspruch 4 oder 5, **dadurch ge-**

**kennzeichnet, daß** das erste und das zweite Verbindungsglied und der erste und der zweite Kurbelarm sich im wesentlichen in einer gemeinsamen Ebene befinden und im wesentlichen ein Viereck bilden, dessen Diagonalen im wesentlichen senkrecht sind, wobei die Diagonale, die die Scheitelpunkte des Vierecks verbindet, die sich bei der Hülse (10) und bei den Basismitteln (21) befinden, mit der Achse (12) der Durchgangsbohrung (11) im wesentlichen zusammenfällt.

7. Vorrichtung nach einem der Ansprüche 1 bis 6, **dadurch gekennzeichnet, daß** wenigstens zwei der folgenden Elemente aus dem gleichen Werkstoff hergestellt sind: Hülse (10); Basismittel (21); Verbindungsglied (13, 14); Kurbelarm (22, 23); Schamiermittel (19, 20); und frei schwenkbare Anbringungsmittel (28, 29, 30, 31).

8. Vorrichtung nach Anspruch 7, **dadurch gekennzeichnet, daß** die wenigstens zwei Elemente durch Gießen hergestellt sind.

9. Vorrichtung nach Anspruch 8, **dadurch gekennzeichnet, daß** der Werkstoff Kunststoff ist.

10. Vorrichtung nach einem der Ansprüche 1 bis 9, **dadurch gekennzeichnet, daß** die Länge "$L_1$" des Verbindungsgliedes und die Länge "$l_1$" des Kurbelarms in der Weise bestimmt sind, daß die Summe $L_1 + l_1$ und die Summe $L_1 + l_1 + M$, wobei "M" die Länge der Hülse (10) repräsentiert, die Länge "A" der zu schützenden Nadel (1), die zwischen ihrem spitzen Ende (2) und ihrem Basisende (3) gemessen wird, umgreifen.

11. Vorrichtung nach einem der Ansprüche 4 bis 10, **dadurch gekennzeichnet, daß** sie ferner ein Gehäuse (70) aufweist, das aus einem elastischen Werkstoff hergestellt ist und unter Spannung jene Baueinheit umgibt, die gebildet ist durch die Hülse (10), das erste und das zweite Verbindungsglied (13, 14), das erste und das zweite Scharniermittel (19, 20), den ersten und den zweiten Kurbelarm (22, 23), die ersten und die zweiten Mittel (28-31) zum schwenkbaren Anbringen des ersten und des zweiten Endes des ersten und des zweiten Kurbelarms an den jeweiligen zweiten Enden (17, 18) des ersten und des zweiten Verbindungsgliedes (13, 14) bzw. an den Basismitteln (21) und wenigstens einen Abschnitt der Basismittel (21).

12. Vorrichtung nach Anspruch 11, **dadurch gekennzeichnet, daß** der elastische Werkstoff, aus dem das Gehäuse (70) hergestellt ist, lichtdurchlässig ist.

13. Vorrichtung nach Anspruch 11 oder 12, **dadurch**

**gekennzeichnet, daß** sie ferner einen Beutel (71), der aus einem nicht dehnbaren Werkstoff hergestellt ist, eine gegebene Substanz enthält und bei einem gegebenen Zug zerreißen kann, und Mittel (72, 73) zum Befestigen des Beutels (71) und des Gehäuses (70) im wesentlichen an zwei gegenüberliegenden Punkten (74, 75) der Innenwand des Gehäuses (70) umfaßt, wobei die beiden Punkte sich im wesentlichen gegenüber den ersten Mitteln (28, 29) zum frei schwenkbaren Anbringen der ersten Enden des ersten und des zweiten Kurbelarms an den zweiten Enden des ersten bzw. des zweiten Verbindungsgliedes befinden.

14. Vorrichtung nach Anspruch 13, **dadurch gekennzeichnet, daß** die in dem Beutel (71) enthaltene Substanz wenigstens eine der folgenden Eigenschaften aufweist: geeignet für die Absorption wenigstens eines Teils des sichtbaren Lichts, geeignet zum Härten, geeignet zum Sterilisieren.

15. Vorrichtung zum Schützen und Neutralisieren einer Nadel (1, 201) für medizinischen Gebrauch oder dergleichen nach einem der Ansprüche 1 bis 14, wobei die Nadel ein spitzes Ende (2, 202) und ein Basisende (203) besitzt, wobei die Vorrichtung umfaßt:

eine Hülse (204) mit einer Durchgangsbohrung (205), die einen ersten und einen zweiten Auslaß (206, 207) besitzt, auf einer gegebenen Achse (208) definiert ist und einen Querschnitt besitzt, der nicht kleiner als jener der Nadel (201) ist, wobei die Nadel durch die Bohrung gleiten kann;
eine Basis (209), die an dem Basisende (203) der Nadel (201) befestigt ist; und
elastische Verbindungsgliedmittel (210), die die Hülse (204) mit der Basis (209) verbinden, wobei die elastischen Verbindungsgliedmittel der Hülse (204) ermöglichen, längs der Nadel (201) zu gleiten, wobei die Nadel durch die Bohrung (205) der Hülse und durch deren ersten Auslaß (206) verläuft, wobei die Hülse zwei Extrempositionen einnehmen kann:

- * eine erste Position, in der die Hülse das spitze Ende (202) der Nadel umgibt, wobei sich das spitze Ende in einem gegebenen Abstand von dem zweiten Auslaß (207) der Durchgangsbohrung (205) befindet; und
- * eine zweite Position, in der die Fläche (211) der Hülse (204), die den ersten Auslaß (206) der Durchgangsbohrung enthält, in der Nähe der Basis (209) positioniert ist;

wobei die Vorrichtung **dadurch gekennzeichnet ist, daß** sie ferner umfaßt:

einen ersten Kanalabschnitt (212), der in der Hülse (204) ausgebildet ist und die Durchgangsbohrung (205) in ihrem Abschnitt schneidet, der zwischen ihrem zweiten Auslaß (207) und dem spitzen Ende (202) der Nadel (201) liegt, wenn sich die Hülse (204) in ihrer ersten Position befindet;
einen Verschluß (213), der im ersten Kanalabschnitt (212) gleitend angebracht ist und eine erste Position und eine zweite Position einnehmen kann, wobei die erste Position jene ist, in der er sich nicht in der Durchgangsbohrung (205) befindet, und die zweite Position jene ist, in der er sich in der Durchgangsbohrung befindet; und
Mittel (220) zum Ausüben eines Schubs auf den Verschluß (213), wenn sich die Hülse (204) der Basis (209) annähert, wenn sie sich aus ihrer ersten Position in ihre zweite Position bewegt.

16. Vorrichtung nach Anspruch 15, **dadurch gekennzeichnet, daß** die Mittel (220), die auf den Verschluß dann, wenn sich die Hülse der Basis annähert, wenn sie sich aus ihrer ersten Position in ihre zweite Position bewegt, einen Schub ausüben, gebildet sind durch:

einen zweiten Kanalabschnitt (221), der in der Hülse (204) in Kontinuität mit dem ersten Kanalabschnitt (212) ausgebildet ist und über eine Auslaßöffnung (222) in der gleichen Fläche (211) der Hülse, in der der erste Auslaß (206) der Durchgangsbohrung (205) vorhanden ist, mündet;
eine flexible Stange (223), die zu einer gekrümmten Bogenform vorgeformt ist und in dem zweiten Kanalabschnitt (221) in der Weise gleitend angebracht ist, daß ein erstes Ende (224) hiervon mit dem Verschluß (213) verbunden ist und ein zweites Ende (225) hiervon aus der Auslaßöffnung (222) des zweiten Kanalabschnitts um einen Betrag vorsteht, der nicht kleiner als die Strekke ist, die der Verschluß (213) zurücklegen muß, um sich aus seiner ersten Position in seine zweite Position zu bewegen; und
einen Freigabehohlraum (226) in der Nähe des zweiten Kanalabschnitts (221) und in Verbindung mit dem zweiten Kanalabschnitt, wobei der Freigabehohlraum so entworfen ist, daß sich die flexible Stange (223) dann, wenn der Verschluß (213) in seiner ersten Position gehalten wird, durch Biegen verformen kann, um seitlich in den Freigabehohlraum (226) einzudringen, wenn sich die Fläche (211) der Hülse, in der sich der erste Auslaß (206) der Durchgangsbohrung (205) befindet, der Basis (209) annähert.

**17.** Vorrichtung nach Anspruch 16, **dadurch gekennzeichnet, daß** sie ferner Mittel (227) umfaßt, die die Position des zweiten Endes (225) der flexiblen Stange (223) verriegeln, wenn die Stange dazu veranlaßt wird, in den zweiten Kanalabschnitt (221) einzutreten.

**18.** Vorrichtung nach Anspruch 17, **dadurch gekennzeichnet, daß** die Mittel (227), die die Position des zweiten Endes (225) der flexiblen Stange (223) dann, wenn diese in den zweiten Kanalabschnitt (221) zurückgezogen ist, verriegeln, durch wenigstens einen Widerhaken (228), der an der flexiblen Stange (223) befestigt ist, und ein Gehäuse (229), das zu dem Widerhaken (228) komplementär ist und in der Wand des zweiten Kanalabschnitts (221) ausgebildet ist, gebildet sind.

**19.** Vorrichtung nach einem der Ansprüche 15 bis 18, **dadurch gekennzeichnet, daß** der erste Kanalabschnitt (212) wenigstens einen ersten Teil (231) und einen zweiten Teil (232) besitzt, die beiderseits der Durchgangsbohrung (205) ausgebildet sind, wobei sich der erste Teil (231) des ersten Kanalabschnitts in einer Linie mit dem zweiten Kanalabschnitt (221) befindet, wobei der Verschluß (213) in dem ersten Teil (231) des ersten Kanalabschnitts (212) vollständig enthalten ist, wenn er sich in seiner ersten Position befindet, und dadurch, daß die Vorrichtung ferner eine im wesentlichen U-förmige Gabel (233), die an dem Verschluß (213) befestigt ist, enthält, wobei die zwei Schenkel (234, 235) der Gabel voneinander um eine Strecke beabstandet sind, die nicht kleiner als der Durchmesser der Nadel (201) ist, wobei die Gabel (233) in der Weise geformt ist, daß sich der Spalt (236), der zwischen den beiden Schenkeln (234, 235) definiert ist, dann, wenn sich der Verschluß (213) in seiner ersten Position befindet, auf der Achse (208) der Durchgangsbohrung (205) befindet und die beiden Schenkel (234, 235) sich wenigstens teilweise in den zweiten Teil (232) des ersten Kanalabschnitts (212) erstrecken.

**20.** Vorrichtung nach Anspruch 19, **dadurch gekennzeichnet, daß** die flexible Stange (223), der Verschluß (213) und die Gabel (233) als einziges Teil ausgebildet sind.

**21.** Vorrichtung nach Anspruch 20, **dadurch gekennzeichnet, daß** das Teil aus Kunststoff durch Gießen hergestellt ist.

**22.** Vorrichtung nach einem der Ansprüche 16 bis 21, **dadurch gekennzeichnet, daß** die beiden Kanalabschnitte (212, 221) in einer Ebene (240) enthalten sind, die außerdem die Achse (208) der Durchgangsbohrung (205) enthält, wobei die Hülse (204) aus zwei Schalen (241, 242) hergestellt ist, die so beschaffen sind, daß sie um die Ebene (40), die die beiden Kanalabschnitte (212, 221) enthält, zusammengefügt sind.

**23.** Vorrichtung nach einem der vorhergehenden Ansprüche, **dadurch gekennzeichnet, daß** die Basismittel (21, 209) zwei erste und zweite Ringe (301-302, 311-312), wobei der erste Ring (301, 331) das untere Ende (3, 203) der Nadel aufnimmt, und Mittel (320), die die beiden Ringe durch Schwachpunkte miteinander verbinden, umfassen.

**24.** Vorrichtung nach Anspruch 23, **dadurch gekennzeichnet, daß** jeder der zwei Ringe eine Öffnung (321, 322) aufweist, wobei die beiden Öffnungen so verwirklicht sind, daß sie dann, wenn die zwei Ringe miteinander verbunden sind, den Buchsenabschnitt einer Steck-Buchsen-Verbindung bilden, der mit dem komplementären Steckabschnitt, der durch einen Endabschnitt (323) einer Spritze (325) gebildet ist, zusammenwirken kann, wobei die Gesamttiefe dieser zwei Öffnungen dann, wenn die beiden Ringe miteinander verbunden sind, geringer als die Höhe des Endabschnitts der Spritze ist.

**25.** Vorrichtung nach einem der Ansprüche 23 und 24, **dadurch gekennzeichnet, daß** sie ferner einen Entnahmeverhinderungsring (330) enthält, der sich an der Wand (331) wenigstens einer der zwei Öffnungen befindet.

**Claims**

**1.** A device for protecting and neutralizing a needle (1, 201) for medical use or the like, the needle having a sharp end (2, 202) and a base end (3, 203), the device comprising :

a sleeve (10, 204) having a through bore (11, 206) defined on a given axis (12, 208), said through bore being of a section that is not less than that of the needle to be protected; a first link (13, 113, 14, 114) having first and second ends (15, 115, 16, 116, 17, 117, 18, 118), said link being of a length "$L_1$" defined between said two ends; base means (21, 209) suitable for receiving the base end (3, 203) of the needle to be protected (1, 201); a first crank arm (22, 122, 23, 123), said first crank arm being defined between first and second ends (24, 124, 25, 125 - 26, 126, 27, 127), said crank arm being of a length "$l_1$" defined between its two ends, the length "$l_1$" of the first crank arm being no greater than the length "$L_1$"

of the first link;

first means (28, 128, 29, 129, 30, 130, 31, 131) for mounting each of said first and second ends of the first crank arm to pivot freely respectively on the second end (17, 18) of the first link and on the base means (21, 209), and

first resilient return hinge means (19, 20) for connecting the first end (15, 16) of the first link to the sleeve (10), and

means (50) to lock said link and said crank arm relative to each other in a blocking position (III), thus protecting the sharp end of the needle in the sleeve,

**characterized by** the fact that said first hinge means (19, 20) are organized so that said first link takes up an initial defined equilibrium position (I) on a direction that makes an acute angle ($\alpha$) with the axis of the through bore, so that the sleeve (10) protects the sharp end (2, 202).

2. A device according to claim 1, **characterized by** the fact that it further includes strut means (40) connecting said link (13, 14) to said crank arm (22, 23) when they are in a first given position, said strut means including a weak point (41) making it possible, on application of a given force, to break said strut means at said weak point.

3. A device according to claim 1 or 2, **characterized by** the fact that said means (50) to lock said link and said crank arm relative to each other in a second given position (Figure 5) are snap-fastening means.

4. A device according to any one of claims 1 to 3, **characterized by** the fact that it further includes:

a second link (13, 14) having first and second ends (15, 16 - 17, 18), said second link being of a length "$L_2$" defined between said two ends; second resilient return hinge means (19, 20) for connecting the first end (15, 16) of the second link to the sleeve (10), said second hinge means being organized so that said second link takes up a defined equilibrium position on a direction that makes an acute angle ($\alpha'$) with the axis of the through bore; a second crank arm (22, 23), said second crank arm being defined between first and second ends (24, 25 - 26, 27), said second crank arm being of a length "$l_2$" defined between its two ends, the length "$l_2$" of the second crank arm being no greater than the length "$L_2$" of the second link; and second means (28-31) for mounting each of said first and second ends of the second crank arm to pivot freely respectively on the second end (13, 14) of the second link and on the base means (21).

5. A device according to claim 4, **characterized by** the fact that the lengths "$L_1$" and "$L_2$" are substantially equal to a common value "L" and that the lengths "$l_1$" and "$l_2$" are substantially equal to a common value "l".

6. A device according to claim 4 or 5, **characterized by** the fact that the first and second links and the first and second crank arms are situated substantially in a common plane and form substantially a quadrilateral whose diagonals are substantially perpendicular, the diagonal interconnecting the vertices of the quadrilateral situated respectively at the sleeve (10) and at the base means (21) coinciding substantially with the axis (12) of the through bore (11).

7. A device according to any one of claims 1 to 6, **characterized by** the fact that at least two of the following elements are made of the same material: sleeve (10); base means (21); link (13, 14); crank arm (22, 23); hinge means (19, 20); and freely pivoting mounting means (28, 29, 30, 31).

8. A device according to claim 7, **characterized by** the fact that said at least two elements are made by molding.

9. A device according to claim 8, **characterized by** the fact that the material is a plastics material.

10. A device according to any one of claims 1 to 9, **characterized by** the fact that the length "$L_1$" of the link and the length "$l_1$" of the crank arm are determined in such a manner that the sum $L_1 + l_1$ and the sum $L_1 + l_1 + M$, where "M" represents the length of the sleeve (10), bracket the length "A" of the needle to be protected (1) as measured between its sharp end (2) and its base end (3).

11. A device according to any one of claims 4 to 10, **characterized by** the fact that it further includes a casing (70) made of a resilient material surrounding under tension the assembly constituted by the sleeve (10), the first and second links (13, 14), the first and second hinge means (19, 20), the first and second crank arms (22, 23), the first and second means (28-31) for mounting the first and second ends of the first and second crank arms to pivot respectively on the second ends (17, 18) of the first and second links (13, 14) and on the base means (21), and at least a portion of the base means (21).

12. A device according to claim 11, **characterized by** the fact that the resilient material from which the casing (70) is made is transparent.

13. A device according to claim 11 or 12, **characterized by** the fact that it further includes a sachet (71) made of a non-stretch material, the sachet (71) containing a given substance and being capable of tearing under a given traction, and means (72, 73) for securing the sachet (71) and the casing (70) substantially at two opposite points (74, 75) of the inside wall of the casing (70), the two said points being situated substantially facing the first means (28, 29) for mounting the first ends of the first and second crank arms to pivot freely on the second ends of the first and second links.

14. A device according to claim 13, **characterized by** the fact that the substance contained in the sachet (71) presents at least one of the following properties: being suitable for absorbing at least a portion of visible light, being suitable for hardening, being suitable for sterilizing.

15. A device for protecting and neutralizing a needle (1, 201) for medical use or the like according to any one of claims 1 to 14, the needle having a sharp end (2, 202) and a base end (203), the device comprising:

   a sleeve (204) having a through bore (205), said through bore having first and second outlets (206, 207), said through bore being defined on a given axis (208) and having a cross-section that is not less than that of the needle (201), the needle being suitable for sliding through said bore;
   a base (209) secured to the base end (203) of the needle (201); and
   resilient link means (210) connecting the sleeve (204) to the base (209), said resilient link means enabling the sleeve (204) to slide along the needle (201), the needle passing through the bore (205) of the sleeve via the first outlet (206) thereof, said sleeve being suitable for taking up two extreme positions:

   * a first position in which the sleeve surrounds the sharp end (202) of the needle, said sharp end being situated at a given distance from the second outlet (207) of the through bore (205); and
   * a second position in which the face (211) of the sleeve (204) that includes the first outlet (206) of the through bore is positioned adjacent to the base (209);

   the device being **characterized by** the fact that it further comprises:

   a first channel portion (212) made in the sleeve (204) and intersecting the through bore (205) in its portion lying between its second outlet (207) and the sharp end (202) of the needle (201) when the sleeve (204) is in its first position;
   a shutter (213) slidably mounted in the first channel portion (212), said shutter being suitable for taking up a first position and a second position, the first position being one in which it is not situated in the through bore (205), and the second position being one in which it is situated in the through bore; and
   means (220) for applying thrust on said shutter (213) when the sleeve (204) comes close to the base (209) on passing from its first position to its second position

16. A device according to claim 15, **characterized by** the fact that the means (220) for applying thrust on said shutter when the sleeve comes close to the base on passing from its first position to its second position are constituted by:

   a second channel portion (221) made in the sleeve (204) in continuity with the first channel portion (212) and opening out via an outlet orifice (222) in the same face (211) of the sleeve as has the first outlet (206) of the through bore (205);
   a flexible rod (223) preformed into an arcuate bow shape and slidably mounted in said second channel portion (221) in such a manner that a first end (224) thereof is associated with the shutter (213), and a second end (225) thereof emerges from the outlet orifice (222) of the second channel portion by an amount that is not less than the distance the shutter (213) needs to travel in order to pass from its first position to its second position; and
   a release cavity (226) adjacent the second channel portion (221) and in communication with said second channel portion, the release cavity being designed so that, when the shutter (213) is held in its first position, the flexible rod (223) can deform in bending to penetrate laterally into said release cavity (226) when the face (211) of the sleeve having the first outlet (206) of the through bore (205) comes close to the base (209).

17. A device according to claim 16, **characterized by** the fact that it further includes means (227) for locking the position of the second end (225) of the flexible rod (223) when said rod is caused to enter into the second channel portion (221).

18. A device according to claim 17, **characterized by** the fact that the means (227) for locking the position of the second end (225) of the flexible rod (223) when it is retracted into the second channel portion (221) are constituted by at least one barb (228) secured to the flexible rod (223) and a housing (229) complementary to the barb (228) formed in the wall of the second channel portion (221).

19. A device according to any one of claims 15 to 18, **characterized by** the fact that the first channel portion (212) has at least a first part (231) and a second part (232) formed on either side of the through bore (205), the first part (231) of the first channel portion being in line with the second channel portion (221), the shutter (213) being contained completely within said first part (231) of the first channel portion (212) when it is in its first position, and by the fact that the device further includes a substantially U-shaped fork (233) secured to the shutter (213), the two limbs (234, 235) of the fork being spaced apart from each other by a distance of not less than the diameter of the needle (201), said fork (233) being shaped in such a manner that, when the shutter (213) is in its first position, the gap (236) defined between the two limbs (234, 235) thereof is situated on the axis (208) of the through bore (205) and the two limbs (234, 235) extend at least in part into the second part (232) of the first channel portion (212).

20. A device according to claim 19, **characterized by** the fact that the flexible rod (223), the shutter (213), and the fork (233) are made as a single piece.

21. A device according to claim 20, **characterized by** the fact that said piece is made of plastics material by molding.

22. A device according to any one of claims 16 to 21, **characterized by** the fact that the two channel portions (212, 221) are contained in a plane (240) that also contains the axis (208) of the through bore (205), the sleeve (204) being made as two half-shells (241, 242) organized to be assembled together about said plane (40) containing the two channel portions (212, 221).

23. A device according to any one of previous claims, **characterized by** the fact that the base means (21, 209) comprises two first and second rings (301-302, 311-312), the first ring (301, 331) receiving the low end (3, 203) of the needle, and means (320) for connecting said two rings between them by weak points.

24. A device according to claim 23, **characterized by** the fact that each of the two rings comprises an opening (321, 322), the two openings being realized to form, when the two rings are connected between them, the female part of a male-female jointing able to cooperate with the complementary male part constituted by an end-part (323) of a syringe (325), the total depth of these two openings when the two rings are connected between them, being lower than the height of end-part of the syringe.

25. A device according to any one of claims 23 and 24, **characterized by** the fact that it further includes a not-withdrawal ring (330) located on the wall (331) of at least one of the two openings.

fig.1

fig.4

fig. 2

fig.3

fig.5

fig. 6

fig. 7

fig.8

fig.9

fig.10

_fig.11_

_fig.12_